## Europäisches Patentamt
## European Patent Office
## Office européen des brevets

(19)

(11) Veröffentlichungsnummer: **0 158 194 B1**

(12) **EUROPÄISCHE PATENTSCHRIFT**

(45) Veröffentlichungstag der Patentschrift: 05.06.91

(21) Anmeldenummer: 85103420.7

(22) Anmeldetag: 22.03.85

(51) Int. Cl.5 **C12P 13/00, C12N 1 20,** //(C12P13/00,C12R1:01), (C12N1/20,C12R1:01)

(54) Verfahren zur Herstellung von L-Carnitin auf mikrobiologischem Weg.

(30) Priorität: 29.03.84 CH 1600/84

(43) Veröffentlichungstag der Anmeldung:
16.10.85 Patentblatt 85/42

(45) Bekanntmachung des Hinweises auf die Patenterteilung:
05.06.91 Patentblatt 91/23

(84) Benannte Vertragsstaaten:
AT BE CH DE FR GB IT LI LU NL SE

(56) Entgegenhaltungen:
EP-A- 0 122 794
EP-A- 0 148 132
GB-A- 2 078 742

(73) Patentinhaber: LONZA AG

Gampel/Wallis(CH)

(72) Erfinder: Kulla, Hans, Dr.
Wildi
CH-Visp (Kanton Wallis)(CH)
Erfinder: Lehky, Pavel, Dr.
Dammweg 13
CH-Naters (Kanton Wallis)(CH)

(74) Vertreter: Weinhold, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
W-8000 München 40(DE)

## Beschreibung

Es ist bekannt, L-Carnitin aus γ-Butyrobetain herzustellen. Das γ-Butyrobetain wird in Gegenwart von Natrium-2-oxoglutarat, einem reduzierenden Agens, einer Eisenionenquelle und einem Luftsauerstoff als Hydroxylgruppendonor mit einem Hydroxylase-Enzym, freigesetzt aus Sporen von Neurospora crassa, in Berührung gebracht (US-PS 4 371 618). Dieses Verfahren weist den Nachteil auf, eine Vielzahl von Cofaktoren zu benötigen. So werden in der Reaktion stöchiometrische Mengen 2-Oxoglutarat oxidativ zu Succinat decarboxyliert. $Fe^{2+}$ wird als $O_2$-Aktivator benötigt, Ascorbat, um das Eisen-Ion in der reduzierten Form zu halten, und Katalase, um das in Spuren entstehende, schädliche $H_2O_2$ zu zerstören.

Lindstedt et al, (Biochemistry 6, 1262-1270 (1967) "The Formation and Degradation of Carnitin in Pseudomonas") isolierten einen Mikroorganismus der Gattung Pseudomonas, der mit γ-Butyrobetain als C- und N-Quelle wächst. Die erste Reaktion des Abbauweges war die Hydroxylierung des γ-Butyrobetains zu L-Carnitin, wobei das intermediär entstehende L-Carnitin vollständig zu $CO_2$, $H_2O$ und $NH_3$ weiterkatabolisiert wurde.

Auch diese, aus Bakterien gewonnene Hydroxylase hätte, wenn sie für die L-Carnitin-Produktion eingesetzt werden würde, die geschilderten nachteiligen Cofaktor-Bedürfnisse (Lindstedt et al, Biochemistry 16, 2181- 2188 (1977) "Purification and Propertiesof γ-Butyrobetaine Hydroxylase from Pseudomonas sp. $\overline{AK}$ 1").

Der Erfindung liegt die Aufgabe zugrunde, einen neuen Typ von Mikroorganismen zu finden, der diese Nachteile der bekannten Verfahren nicht aufweist und es ermöglicht, auf einfache Weise nicht das Razemat, sondern enantio-selektiv L-Carnitin aus Crotonobetain, Butyrobetain oder Gemischen davon herzustellen.

Im Unterschied zu den im Stand der Technik bekannten Systemen verwenden unsere Mikroorganismen das $H_2O$ und nicht das $O_2$ als Hydroxylgruppendonor, wie durch eigene Untersuchungen unter Verwendung von $H_2{}^{18}O$ und $^{18}O_2$ festgestellt werden konnte.

Die erfindungsgemässen Mikroorganismen sind befähigt, aus Crotonobetain und/oder γ-Butyrobetain L-Carnitin zu produzieren und letzteres nicht zu katabolisieren.

Wie eine vergleichende Untersuchung an Bodenproben aus vier Kontinenten zeigte, sind Mikroorganismen, die Butyrobetain und Crotonobetain via L-Carnitin katabolisieren, ubiquitär. Auch aus dem Belebtschlamm von Abwasserreinigungsanlagen gelingt die Isolierung. Potentiell kommen alle diese Stämme als L-Carnitin-Produzenten in Frage, wenn sie nach dem unten angegebenen, erfindungsgemässen Prinzip mutiert werden.

Solche Mutanten sind erhältlich durch folgende Selektionsmethode:

a) Mikroorganismen, die mit Betain, γ-Butyrobetain, Crotonobetain und L-Carnitin als C- und N-Quelle wachsen, werden auf übliche Weise mutiert.

b) Aus der durch Züchtung erhaltenen Kultur von mutierten Mikroorganismen werden jene selektioniert, die stabil sind, L-Carnitin nicht katabolisieren und nicht auf L-Carnitin, Crotonobetain, γ-Butyrobetain wachsen, wohl aber mit Betain.

Vorzugsweise werden folgend auf Selektionsschritt b) jene Mikroorganismen ausgewählt, die L-Carnitin ausscheiden und nicht auf L-Carnitin, Crotonobetain, γ-Butyrobetain wachsen, wohl aber mit Betain.

Zweckmässig werden die mutierten Mikroorganismen in einem Betain-Medium weiterkultiviert und diese weiterkultivierten Mikroorganismen zur Durchführung des Selektionsschrittes b) vorzugsweise in einem L-Carnitin-Medium weitergezüchtet. Die Züchtung von mit Betain, γ-Butyrobetain, Crotonobetain und L-Carnitin als C- und N-Quelle wachsenden Stämme wird zweckmässig so durchgeführt, dass man aus Bakteriengemischen durch Beimpfung von Crotonobetain-Nährlösungen Mischkulturen erzeugt und aus denen unter Zuhilfenahme traditioneller mikrobiologischer Techniken dann Reinkulturen von Crotonobetain abbauenden Mikroorganismen anlegt.

Die Mutation einer solchen Kultur, die mit Betain, γ-Butyrobetain, Crotonobetain und L-Carnitin als C- und N-Quelle wächst, kann nach bekannten Methoden durchgeführt werden [J.H. Miller, Experiments in Molecular Genetics, Cold Spring Harbor Laboratory, 1972].

Zweckmässig anwendbare Methoden zur Herstellung stabiler Mutanten sind die Frame-Shift-Methode, Deletionsmethode oder die Transposon-Insertionsmethode.

Die so mutierten Mikroorganismen werden dann nach der Weiterkultivierung in einem Betain-Medium und der Ueberführung in ein L-Carnitin-Medium dem Selektionsschritt b) unterzogen, wo mittels bekannten " counter-selektionierenden Agenzien" [P.Gerhardt et al (eds.), Manual of Methods for General Bacteriology, Am.Soc. for Microbiology, 1981] jene Mikroorganismen selektioniert werden, die stabil L-Carnitin nicht katabolisieren und nicht auf L-Carnitin, Crotonobetain, γ-Butyrobetain wachsen, wohl aber mit Betain.

Ein bevorzugter Mikroorganismus, der mit Betain, γ-Butyrobetain, Crotonobetain, L-Carnitin als C- und N-Quelle wächst, ist der Stamm HK 4 (DSM Nr. 2938) und dessen Deszendenten und Mutanten.

Dieser Stamm wurde am 3.3.1984 bei der Deutschen Sammlung von Mikroorganismen (DSM). Gesellschaft für Biotechnologische Forschung mbH, Griesebachstrasse 8, 4300 Göttingen, Bundesrepublik Deutschland, unter der Nr. DSM 2938 hinterlegt.

Wissenschaftliche Beschreibung des Stammes HK 4 (DSM Nr. 2938)

| | | | |
|---|---|---|---|
| Zellform | Stäbchen, z.T.pleomorph | Phenylalanindeaminase | − |
| | | Ornithindecarboxylase | − |
| Länge μm | 1-2 | | |
| Breite μm | 0,5-0,8 | | |
| Beweglichkeit | + | $H_2S$ | − |
| Geisseln | peritrich | Voges-Proskauer | − |
| Gram-Reaktion | − | Indol | − |
| Sporen | − | Nitrit aus Nitrat | + |
| Bildung von Poly-β-hydro- | − | Denitrifikation | + |
| xybutyrat | | Levanbildung | − |
| Oxidase | + | Lecithinase | − |
| Catalase | + | Urease | + |
| Wachstum | | Abbau von | |
| anaerob | − | Stärke | − |
| 37°C | + | Gelatine | − |
| 41°C | − | Casein | − |
| pH 5,6 | − | Tyrosin | − |
| Mac-Conkey-Agar | + | Tween 80 | − |
| SS-Agar | − | DNA | + |
| Cetrimid-Agar | − | Aesculin | + |
| Pigmentbildung | | Substratverwertung | |
| nicht diffundierend | − | Acetat | − |
| diffundierend | − | Citrat | − |
| fluoreszierend | − | Malonat | − |
| Säurebildung (OF-Test) aus | | Glycin | − |
| Glucose aerob | − | Norleucin | − |
| anaerob | − | Xylose | + |
| Fructose aerob | − | Fructose | + |
| ASS Glucose | + | Glucose | + |
| Xylose | + | Autotrophes Wachstum | − |
| Trehalose | + | mit $H_2$ | |
| Ethanol | − | 3-Ketolactose | − |

| | | | |
|---|---|---|---|
| Gasbildung aus Glucose | − | Wachstum | |
| ONPG | + | Betain | + |
| Argininindihydrolase | − | L-Carnitin | + |
| Lysindecarboxylase | − | γ-Butyrobetain | + |
| | | Crotonobetain | + |

Ein bevorzugter Mikroorganismus, der aus dem vorstehend beschriebenen Mikroorganismus mutiert und selektioniert wurde, stabil ist, L-Carnitin nicht katabolisiert, sondern ausscheidet und nicht auf L-Carnitin, Crotonobetain und γ-Butyrobetain wächst, wohl aber auf Betain wächst, ist der HK 13.

Dieser genannte Stamm wurde am 23.1.1984 bei der Deutschen Sammlung von Mikroorganismen (DSM), Gesellschaft für Biotechnologische Forschung mbH, Griesebachstrasse 8, 4300 Göttingen, Bundesrepublik Deutschland, unter der Nr. DSM 2903 deponiert.

## Wissenschaftliche Beschreibung des Stammes HK 13 (DSM Nr.2903)

| | | | |
|---|---|---|---|
| Zellform | Stäbchen z.T.pleomorph | Phenylalanindeaminase | − |
| Länge μm | 1-2 | Ornithindecarboxylase | − |
| Breite μm | 0,5-0,8 | | |
| Beweglichkeit | + | $H_2S$ | − |
| Geisseln | peritrich | Voges-Proskauer | − |
| Gram-Reaktion | − | Indol | − |
| Sporen | − | Nitrit aus Nitrat | + |
| Bildung von Poly-β-hydroxybutyrat | − | Denitrifikation | + |
| | | Levanbildung | − |
| Oxidase | + | Lecithinase | − |
| Catalase | + | Urease | + |

| Wachstum | | Abbau von | |
|---|---|---|---|
| anaerob | – | Stärke | – |
| 37°C | + | Gelatine | – |
| 41°C | – | Casein | – |
| pH 5,6 | – | Tyrosin | – |
| Mac-Conkey-Agar | + | Tween 80 | – |
| SS-Agar | – | DNA | + |
| Cetrimid-Agar | – | Aesculin | + |
| Pigmentbildung | | Substratverwertung | |
| nicht diffundierend | – | Acetat | – |
| diffundierend | – | Citrat | – |
| fluoreszierend | – | Malonat | – |
| Säurebildung (OF-Test) aus | | Glycin | – |
| Glucose aerob | – | Norleucin | – |
| anaerob | – | Xylose | + |
| Fructose aerob | – | Fructose | + |
| ASS Glucose | + | Glucose | + |
| Xylose | + | Autotrophes Wachstum | – |
| Trehalose | + | mit $H_2$ | |
| Ethanol | – | 3-Ketolactose | – |
| Gasbildung aus Glucose | – | Wachstum | |
| ONPG | + | Betain | + |
| Arginindihydrolase | – | L-Carnitin | – |
| Lysindecarboxylase | – | γ-Butyrobetain | – |
| | | Crotonobetain | – |
| | | L-Glutamat und Crotonbetain | ± |
| | | L-Glutamat und Butyrobetain | ± |
| | | L-Glutamat und L-Carnitin | ± |

Als Beispiel eines Dezendenten des Mikroorganismus HK 13, der stabil ist, L-Carnitin nicht katabolisiert, sondern ausscheidet, nicht auf L-Carnitin, Crotonbetain und γ-Butyrobetain wächst, wohl aber mit Betain, L-Glutamat und Crotonbetain, L-Glutamat und Butyrobetain, L-Glutamat und L-Carnitin wächst, ist der Stamm HK 1331 b.

Er wurde als spontane, gut wachsende Mutanten-Kolonie von der Oberfläche eines mit Agar verfestigten Nährmediums, das L-Glutamat und γ-Butyrobetain enthielt, isoliert.

Dieser genannte Stamm wurde am 8.2.1985 bei der Deutschen Sammlung für Mikroorganismus (DSM), Gesellschaft für Biotechnologische Forschung mbH, Griesebachstrasse 8, 4300 Göttingen BRD, unter der

# EP 0 158 194 B1

Nr. DSM 3225 deponiert.

Wissenschaftliche Beschreibung des Stammes HK 1331 b (DSM Nr. 3225 )

| Zellform | Stäbchen z.T.pleomorph | Phenylalanindeaminase | - |
|---|---|---|---|
| Länge μm | 1-2 | Ornithindecarboxylase | - |
| Breite μm | 0,5-0,8 | | |
| Beweglichkeit | + | $H_2S$ | - |
| Geisseln | peritrich | Voges-Proskauer | - |
| Gram-Reaktion | - | Indol | - |
| Sporen | - | Nitrit aus Nitrat | + |
| Bildung von Poly-β-hydro-xybutyrat | - | Denitrifikation | + |
| | | Levanbildung | - |
| Oxidase | + | Lecithinase | - |
| Catalase | + | Urease | + |
| Wachstum | | Abbau von | |
| anaerob | - | Stärke | - |
| 37°C | + | Gelatine | - |
| 41°C | - | Casein | - |
| pH 5,6 | - | Tyrosin | - |

| | | | |
|---|---|---|---|
| Mac–Conkey-Agar | + | Tween 80 | – |
| SS-Agar | – | DNA | + |
| Cetrimid-Agar | – | Aesculin | + |
| Pigmentbildung | | Substratverwertung | |
| nicht diffundierend | – | Acetat | – |
| diffundierend | – | Citrat | – |
| fluoreszierend | – | Malonat | – |
| Säurebildung (OF-Test) aus | | Glycin | – |
| Glucose aerob | – | Norleucin | – |
| anaerob | – | Xylose | + |
| Fructose aerob | – | Fructose | + |
| ASS Glucose | + | Glucose | + |
| Xylose | + | Autotrophes Wachstum | – |
| Trehalose | + | mit H$_2$ | |
| Ethanol | – | 3-Ketolactose | – |
| Gasbildung aus Glucose | – | Wachstum | |
| ONPG | + | Betain | + |
| Arginindihydrolase | – | L-Carnitin | – |
| Lysindecarboxylase | – | γ-Butyrobetain | – |
| | | Crotonobetain | – |
| | | L-Glutamat und | |
| | | Crotonbetain | + |
| | | L-Glutamat und | |
| | | Butyrobetain | + |
| | | L-Glutamat und | |
| | | L-Carnitin | + |

Das Verfahren zur Herstellung von L-Carnitin wird zweckmässig derart ausgeführt, dass man eine Vorkultur eines Mikroorganismus, vorzugsweise eines Mikroorganismus mit der Bezeichnung HK 13 in einem sterilisierten, vorzugsweise vitaminhaltigen Mineralmedium [Kulla et al. Arch. Microbiol. 135, 1 (1983)] bei 20 bis 40° C, vorzugsweise bei 30° C, bei einem zweckmässigen pH-Wert von 6 bis 8, vorzugsweise 7, während 20 bis 50 Stunden, vorteilhaft während 30 bis 40 Stunden, kultiviert. Diese Vorkultur enthält zweckmässig 0,1 bis 10 Gew.%, vorzugsweise 0.5 bis 5 Gew.%, Cholin, Glutamat, Acetat, Dimethylglycin oder Betain als Wachstumssubstrat. Besonders bevorzugt wird Betain in Mengen von 0.5 bis 5 Gew.% eingesetzt.

Des weiteren ist es in der mikrobiologischen Technik üblich, der Vorkultur auch die umzusetzenden Ausgangsverbindungen γ-Butyrobetain, Crotonobetain oder Gemische davon in Mengen von 0,1 bis 10 Gew.%, vorzugsweise 0,5 bis 5 Gew.%, bezogen auf das Reaktionsmedium, zuzusetzen.

Das γ-Butyrobetain bzw. Crotonobetain kann als Hydrochloridsalz, als freies inneres Salz sowie in Form seiner Derivate vorliegen.

Mit dem nach oben genannten Verfahren hergestellten Vorkulturen können weitere Kulturen beimpft werden.

Diese weiteren Kulturen haben zweckmässig die gleiche Zusammensetzung wie die Vorkulturen.

7

Das umzusetzende Crotonobetain, γ-Butyrobetain oder Gemische davon liegen in einer Konzentration von 0,1 bis 10 Gew.%, vorzugsweise 0,5 bis 5 Gew.%, vor.

Auch die Wachstumssubstrate Cholin, Glutamat, Acetat, Dimethylglycin und Betain werden zweckmässig in den bei der Vorkultur verwendeten Konzentrationen angewendet.

Vorteilhafterweise werden die Kultivierungsbedingungen der weiteren Kultivierung entsprechend den Kultivierungsbedingungen der Vorkultivierung angepasst.

Die Temperaturen bewegen sich also zweckmässig zwischen 20 und 40°C, vorteilhaft bei 30°C und der pH-Wert wird in der Regel zwischen 6 und 8 pH, vorteilhaft bei 7 pH, gehalten.

Eine auf diese Weise durchgeführte Herstellung von L-Carnitin kommt nach 20 bis 30 Stunden zum Stillstand.

Die Konzentration an L-Carnitin ist dann in der Regel der eingesetzten Menge an γ-Butyrobetain oder Crotonobetain äquivalent.

Die Zellen können abzentrifugiert oder abfiltriert und als Impfmaterial für eine neue Kultur verwendet werden.

Auf an sich bekannte Weise (J.P.Vandecasteele, Appl.Environ. Microbiol. 39, 327 [1980]) kann das L-Carnitin mittels Kationen-Austauscherchromatographie aus dem Ueberstand herausgeholt und durch Umkristallisation gereinigt werden.

Das Verfahren zur Herstellung von L-Carnitin kann auch auf kontinuierliche Weise durchgeführt werden, indem man die Zellen in einem Chemostaten bei zweckmässigen Verdünnungsraten von 0,04 bis 0,2 $h^{-1}$, vorzugsweise bei 0,06 bis 0,08 $h^{-1}$, bei den analogen Bedingungen wie bei der Batch-Kultur wachsen lässt.

Beispiel 1

Isolation eines Crotonobetain abbauenden Mikroorganismus:

Mikroorganismen wurden aus Erde mit neutraler Phosphat-Pufferlösung durch Rühren extrahiert, anschliessend grössere Bestandteile durch Filtrierpapier abgetrennt und eine Crotonobetain-Nährlösung mit dem so gewonnenen Bakteriengemisch bis zur leichten Trübung beimpft. Nach 9 Tagen war die Trübung als Mass der Zellkonzentration auf das neunzigfache angestiegen. Crotonobetain war aus der Lösung vollständig verschwunden und Ammonium als Abbauprodukt nachweisbar.

Aus der Mischkultur wurden unter Zuhilfenahme traditioneller mikrobiologischer Techniken (verfestigte Agar-Nährböden) Reinkulturen von Crotonobetain abbauenden Mikroorganismen angelegt. Eine Kultur wurde für das weitere Arbeiten ausgewählt und als HK 4 benannt.

Dieser Stamm wächst auch mit γ-Butyrobetain, L-Carnitin und Betain.

Beispiel 2

Isolation einer stabilen Carnitin-Dehydrogenase negativen Mutante:

Eine solche Mutante sollte das aus γ-Butyrobetain oder Crotonobetain aufgebaute L-Carnitin nicht weiter katabolisieren können und im Idealfall stattdessen ausscheiden.

Eine Kultur von HK 4 wurde mit "Acridin Mutagen ICR 191", 5 Microgramm pro ml, in einem Succinat-Medium vorschriftsgemäss [J.H.Miller, Experiments in Molecular Genetics, Cold Spring Harbor Laboratory, 1972] stabil mutagenisiert, danach liess man die Zellen standardgemäss in "Nutrient Broth" zur Expression der Mutation wachsen, danach wurde in ein Betain-Medium überführt. Mit der ausgewachsenen Kultur wurde ein L-Carnitin-Medium beimpft.

Nach wenigen Stunden erreichte die Kultur logarithmisches Wachstum. Zu diesem Zeitpunkt wurde Penicillin G (15 mg/ml) und D-Cycloserin (0,5 mg/ml) zugegeben [Ornston et al, Biochim. Biophys.Res.Commun. 36, 179 (1969)]. Diese "counterselektionierenden" Agenzien töten nur wachsende Bakterien ab. Die Mutanten, die mit L-Carnitin nicht mehr wachsen können und für die wir uns interessieren, überleben und reichern sich demgemäss relativ an. Nach 30 Stunden war die Lebendzellzahl auf ein Hundertstel zurückgegangen, die Antibiotika wurden weggewaschen und die Kultur wurde in ein Betain-Medium überführt. Nach Wachstum wurden entsprechende Verdünnungen der Kultur auf verfestigtem Nähragar verteilt. Die so vereinzelten Zellen wuchsen zu Kolonien heran und wurden einzeln geprüft.

Die Mutante HK 13 wurde ausgewählt. Sie hat stabil keine Carnitin-Dehydrogenase mehr und wächst demgemäss nicht mehr mit L-Carnitin, γ-Butyrobetain oder Crotonobetain, wohl aber mit Betain. Bei Wachstum auf Betain, Dimethylglycin, Cholin, Glutamat oder Acetat wandelt dieser Stamm Crotonobetain oder γ-Butyrobetain in L-Carnitin um und scheidet dieses aus.

Beispiel 3

Eine 5-l-Vorkultur von HK 13 wurde in einem vitaminhaltigen Mineralmedium [Kulla et al, Arch.Microbiol. 135, 1 (1983)], welches 1 Gew.% Betain und 0,5 Gew.% Crotonobetain-chlorid enthielt, bei 30°C und pH 7.0 während 32 Stunden kultiviert. Hiermit wurde eine Kultur von 15 l gleicher Zusammensetzung beimpft und wie die Vorkultur (30°C, pH 7,0, pO₂ = 3 ppm) 24 Stunden lang kultiviert. Als die Produktion zum Stillstand kam, wurden die Zellen abzentrifugiert und konnten als Impfmaterial für einen neuen Batch verwendet werden. Die Konzentration von L-Carnitin wurde im Ueberstand (19,8 l) durch enzymatische Analyse gemessen.

Der Ueberstand enthielt 4,26 mg L-Carnitin pro ml. Dies entsprach einer Ausbeute von 95,0%, auf die eingesetzte Menge von Crotonobetain-chlorid berechnet.

Edukte oder andere Verunreinigungen konnten im NMR-Spektrum nicht nachgewiesen werden.

Wie beschrieben [J.P.Vandecasteele, Appl.Environ.Microbiol. 39, 327 (1980)] konnte das L-Carnitin mittels Kationen-Austauscher-Chromatographie aus dem Ueberstand herausgeholt und durch Umkristallisation gereinigt werden.

Beispiel 4

Eine 5-l-Vorkultur von HK 13 wurde in einem vitaminhaltigen Mineralmedium (gemäss Beispiel 1), welches 1% Cholin und 0,6% γ-Butyrobetain-chlorid enthielt, bei pH 7,0 und 30°C 32 Stunden lang kultiviert. Mit dieser Vorkultur wurde eine Kultur von 15 l Medium gleicher Zusammensetzung beimpft und unter den gleichen Bedingungen wie in Beispiel 1 gezüchtet.

Als die Produktion nach ca. 30 Stunden zum Stillstand kam, wurden die Zellen durch Mikrofiltration (Amicon Hollow-fiber cartridge) abgetrennt. Diese Zellmasse konnte zur weiteren Produktion von L-Carnitin benutzt werden. Die Konzentration von L-Carnitin wurde im Filtrat (19,6 l) enzymatisch bestimmt. Das Filtrat enthielt 5,3 mg L-Carnitin pro ml. Dies entsprach einer analytischen Ausbeute von 97,6%, auf die eingesetzte Menge von γ-Butyrobetain-chlorid berechnet.

Laut NMR-Analyse waren keine Edukte oder andere fremden organischen Nebenprodukte im Filtrat nachweisbar. Deshalb konnte aus der Lösung das L-Carnitin auf bekannte Weise, z.B. mittels azeotroper Destillation (DE-PS 23 00 492) isoliert werden.

Beispiel 5

Ein für kontinuierliche Kultur ausgerüsteter Fermentor, der 1,5 l eines vitaminhaltigen Mineralmediums (gemäss Beispiel 1) mit 1,5% Betain und 1,0% γ-Butyrobetain-chlorid enthielt, wurde mit 150 ml HK 13 Vorkultur gleichen Mediums beimpft. Nach 20 Stunden aerober Züchtung bei 30°C und pH 7,0 war die Kultur ausgewachsen und der kontinuierliche Betrieb konnte mit einer Flussrate von 0,1 l h beginnen. Die dem Fermentor entströmende Kulturlösung wurde in einem auf 4°C gekühlten Gefäss aufgefangen. Die Zellen wurden durch Zentrifugation entfernt.

Laut enzymatischer Analyse enthielt der Ueberstand 8,8 g L-Carnitin pro l Kultur.

Dies entsprach einer analytisch nachgewiesenen Ausbeute von 99,2%, auf die eingesetzte Konzentration des γ-Butyrobetain-chlorids berechnet.

Das feste L-Carnitinchlorid konnte aus der Lösung mittels Ionenchromatographie und Wasserabtrennung isoliert werden.

**Ansprüche**

1. Mikroorganismen, die befähigt sind aus γ-Butyrobetain oder einem Gemisch von γ-Butyrobetain und Crotonobetain L-Carnitin zu produzieren und letzteres nicht zu katabolisieren.

2. Mikroorganismen, die befähigt sind, aus Crotonobetain und/oder γ-Butyrobetain L-Carnitin zu produzieren und letzteres nicht zu katabolisieren, erhältlich nach folgender Selektionsmethode:
   a) Mikroorganismen, die mit Betain, γ-Butyrobetain, Crotonobetain und L-Carnitin als C- und N-Quelle wachsen, werden auf übliche Weise mutiert.
   b) Aus der durch Züchtung erhaltenen Kultur von mutierten Mikroorganismen werden jene selektioniert, die stabil sind, L-Carnitin nicht katabolisieren und nicht auf L-Carnitin, Crotonobetain, γ-Butyrobetain wachsen, wohl aber mit Betain.

3. Mikroorganismen nach Anspruch 2, dadurch gekennzeichnet, dass folgend auf Schritt b) jene Mikroorganismen selektioniert werden, die L-Carnitin ausscheiden und nicht auf L-Carnitin, Crotonobetain, γ-Butyrobetain wachsen, wohl aber mit Betain.

4. Mikroorganismen nach Anspruch 2 oder 3, dadurch gekennzeichnet, dass die in a) mutierten Mikroorganismen in einem Betain-Medium weiterkultiviert werden.

5. Mikroorganismen nach Anspruch 3 bis 4, dadurch gekennzeichnet, dass die im Betain-Medium weitergezüchteten Mikroorganismen zur Durchführung des Selektionsschrittes b) in einem L-Carnitin-Medium gezüchtet werden.

6. Mikroorganismus HK 13 (DSM Nr. 2903) und dessen Deszendenten und Mutanten.

7. Mikroorganismus HK 1331 b (DSM Nr. 3225 ) und dessen Deszendenten und Mutanten.

8. Mikroorganismus HK 4 (DSM Nr. 2938) und dessen Deszendenten und Mutanten.

9. Verfahren zur Herstellung von L-Carnitin, dadurch gekennzeichnet, dass man einen Mikroorganismus nach Ansprüchen 1 bis 7 mit Crotonobetain und/oder γ-Butyrobetain in Gegenwart eines Wachstumssubstrates kultiviert und das angereicherte L-Carnitin isoliert.

10. Verfahren nach Patentanspruch 9, dadurch gekennzeichnet, dass Crotonobetain, γ-Butyrobetain oder die Gemische davon in Mengen von 0,1 bis 10 Gew.%, bezogen auf das Kulturmedium, angewendet werden.

11. Verfahren nach Patentanspruch 9, dadurch gekennzeichent, dass als Wachstumssubstrat Dimethylglycin, Cholin, Glutamat, Acetat und/oder Betain verwendet wird.

12. Verfahren nach Patentanspruch 9, dadurch gekennzeichnet, dass die Wachstumssubstrate in Mengen von 0,1 bis 10 Gew.%, bezogen auf das Kulturmedium, angewendet werden.

## Claims

1. Microorganisms which are able to produce L-carnitine from gamma-butyrobetaine or from a mixture of gamma-butyrobetaine and crotonobetaine and which do not catabolize L-carnitine.

2. Microorganisms which are able to produce L-carnitine from crotonobetaine and/or gamma-butyrobetaine and which do not catabolize L-carnitine, obtainable by the following selection method:
   a) microorganisms which grow with betaine, gamma-butyrobetaine, crotonobetaine and L-carnitine as carbon and nitrogen source are mutated in usual manner;
   b) from the culture of mutated microorganisms obtained by cultivation those microorganisms are selected which are stable, which do not catabolize L-carnitine and which do not grow on L-carnitine, crotonobetaine, gamma-butyrobetaine but do grow with betaine.

3. Microorganisms according to claim 2, characterized in that subsequent to step b) those microorganisms are selected with secrete L-carnitine and which do not grow on L-carnitine, crotonobetaine, gamma-butyrobetaine but do grow with betaine.

4. Microorganisms according to claim 2 or 3, characterized in that the microorganisms mutated in a) are further cultivated in a betaine medium.

5. Microorganisms according to claims 3 to 4, characterized in that the microorganisms which are further cultivated in the betaine medium are cultivated in an L-carnitine medium to effect selection step b).

6. Microorganism HK 13 (DSM No. 2903) and its descendants and mutants.

7. Microorganism HK 1331 b (DSM No. 3225) and its descendants and mutants.

8. Microorganism HK 4 (DSM NO. 2938) and its descendants and mutants.

9. Process for the production of L-carnitine, characterized in that a microorganism according to claims 1 to 7 is cultivated with crotonobetaine and/or butyrobetaine in the presence of a growth substrate and the enriched L-carnitine is isolated.

10. Process according to patent claim 9, characterized in that crotonobetaine. gamma-butyrobetaine or their mixtures are used in amounts of 0.1 to 10 weigh%, based on the culture medium.

11. Process according to patent claim 9, characterized in that as growth substrate dimethylglycine. choline. glutamate, acetate and/or betaine is used.

12. Process according to patent claim 9, characterized in that the growth substrate is used in amounts of 0.1 to 10 weight%, based on the culture medium.

## Revendications

1. Micro-organismes qui sont capables de produire de la L-carnitine à partir de $\gamma$-butyrobétaïne ou d'un mélange de $\gamma$-butyrobétaïne et de crotonobétaïne et non capables de cataboliser la L-carnitine

2. Micro-organismes qui sont capables de produire de la L-carnitine à partir de crotonobétaïne et ou de $\gamma$-butyrobétaïne et non capables de cataboliser la L-carnitine. micro-organismes qui peuvent être obtenus selon la méthode de sélection suivante :
   a) des micro-organismes qui croissent avec la bétaïne, la $\gamma$-butyrobétaïne, la crotonobétaïne et la L-carnitine comme source de C et de N, sont mutés de manière usuelle ;
   b) à partir de la culture obtenue par culture de micro-organismes mutés. on sélectionne ceux qui sont stables, ne catabolisent pas la L-carnitine et ne croîssent pas sur la L-carnitine. la crotonobétaïne, la $\gamma$-butyrobétaïne, mais bien avec la bétaïne.

3. Micro-organismes selon la revendication 2, caractérisés en ce qu'à la suite de l'étape b). on sélectionne les micro-organismes qui libérent de la L-carnitine et ne croîssent pas sur la L-carnitine. la crotonobétaïne, la $\gamma$-butyrobétaïne, mais bien avec la bétaïne.

4. Micro-organismes selon la revendication 2 ou 3. caractérisés en ce que les micro-organismes mutés en a) sont encore cultivés dans un milieu de bétaïne.

5. Micro-organismes selon la revendication 3 à 4. caracrérisés en ce que les micro-organismes encore cultivés dans le milieu de bétaïne sont cultivés dans un milieu de L-carnitine pour effectuer l'étape de sélection b).

6. Le micro-organisme HK 13 (n° DSM 2903) et ses descendants et mutants.

7. Le micro-organisme HK 1331 b (n° DSM 3225) et ses descendants et mutants.

8. Le micro-organisme HK 4 (n° DSM 2938) et ses descendants et mutants.

9. Procédé de préparation de la L-carnitine, caractérisé en ce qu'on cultive un micro-organisme selon les revendications 1 à 7 avec de la crotonobétaïne et ou de la $\gamma$-butyrobétaïne en présence d'un substrat de croissance et en ce qu'on isole la L-carnitine enrichie.

10. Procédé selon la revendication 9, caractérisé en ce qu'on utilise de la crotonobétaïne. de la $\gamma$-butyrobétaïne ou leurs mélanges en des quantités de 0.1 à 10% en poids, par rapport au milieu de culture.

11. Procédé selon la revendication 9, caractérisé en ce qu'on utilise comme substrat de croissance. de la diméthylglycine, de la choline, du glutamate, de l'acétate et ou de la bétaïne.

12. Procédé selon la revendication 9, caractérisé en ce que les substrats de croissance sont utilisés en des quantités de 0,1 à 10% en poids, par rapport au milieu de culture.